# FASCICULE DE BREVET EUROPEEN

(11) **EP 3 027 170 B1**
(45) Date de publication et mention de la délivrance du brevet: **25.04.2018**
(21) Numéro de dépôt: 14759006.1
(22) Date de dépôt: 23.07.2014
(51) Int. Cl.: A61Q 5/00, A61K 8/60, A61K 8/67, A61Q 19/08, A61Q 19/10, A61Q 5/02, A61Q 17/04, A61Q 19/00

(54) **NOUVELLES UTILISATIONS D'ALKYL POLYGLYCOSIDES POUR SOLUBILISER DANS L'EAU DE LA VITAMINE E; COMPOSITIONS LES COMPRENANT**
NEUE VERWENDUNGEN VON ALKYLPOLYGLYKOSIDEN, UM VITAMIN E IN WASSER ZU LÖSEN, ENTSPRECHENDE ZUSAMMENSETZUNGEN
NOVEL USES OF ALKYLPOLYGLYCOSIDES FOR SOLUBILISING VITAMIN E IN WATER, CORRESPONDING COMPOSITIONS

(30) Priorité: 31.07.2013 FR 1357591
(43) Date de publication de la demande: 08.06.2016
(73) Titulaire: Société d'Exploitation de Produits pour les Industries Chimiques SEPPIC, 75321 Paris cedex 07 (FR)
(72) Inventeur: BRETON, Marie-Laure, F-81100 Castres (FR); KERVERDO, Sébastien, F-94300 Vincennes (FR); GARCEL, Stéphanie, F-81100 Castres (FR); GUILBOT, Jerôme, F-81100 Castres (FR); ROLLAND, Hervé, F-81100 Castres (FR)
(74) Mandataire: Laigneau, Amandine
(86) Numéro de dépôt international: PCT/FR2014/051905
(87) Numéro de publication internationale: WO 2015/015092

(56) Documents cités:
- WO-A1-2012/069730
- US-A1- 2003 181 347
- Philippe Lapeyre: "A sustainable solubiliser and co-surfactant for cosmetics", Specialty Chemicals Magazine, juillet 2011 (2011-07), pages 26-27, XP002722546, Extrait de l'Internet: URL:http://www.wheatoleo.com/sites/default /files/july_2011_speciality_chemicals_maga zine.pdf [extrait le 2014-03-31]

## Description

La présente invention concerne l'utilisation de n-heptyle polyglucoside ou de 2-éthyl hexyl polyglucoside pour solubiliser la vitamine E, se présentant soit sous la forme d'un tocophérol, soit sous la forme d'un tocotriénol, dans des compositions aqueuses. L'invention concerne également des compositions aqueuses comprenant de tels alkyl polyglycosides et la vitamine E, leurs utilisations pour le nettoyage, pour la protection et/ou pour le soin de la peau, des cheveux, du cuir chevelu ou des muqueuses, ainsi que leurs utilisations pour des applications pharmaceutiques et alimentaires.

La vitamine E est l'agent antioxydant liposoluble le plus important présent dans le corps des mammifères. Elle est apportée au corps des mammifères par le régime alimentaire, par exemple par l'ingestion de légumes frais, d'huiles végétales, de céréales, de noix, de certaines viandes et de certains produits laitiers. Par vitamine E, on désigne huit formes naturelles :
l'α-tocophérol ou (2R)-2,5,7,8-tétraméthyl-2-[(4R,8R)-4,8,12-triméthyltridécyl]-3,4-dihydrochromèn-6-ol,
le β-tocophérol ou (2*R*)-2,5,8-triméthyl-2-[(4*R*,8*R*)-4,8,12-triméthyltridécyl]-3,4-dihydrochromèn-6-ol,
le γ-tocophérol ou (2*R*)-2,7,8-triméthyl-2-[(4*R*,8*R*)-4,8,12-triméthyltridécyl]-3,4-dihydrochromèn-6-ol,
le δ-tocophérol ou (2*R*)-2,8-diméthyl-2-[(4*R*,8*R*)-4,8,12-triméthyltridécyl]-3,4-dihydrochromèn-6-ol,
l'α-tocotriènol ou (2*R*)-2,5,7,8-tétraméthyl-2-[(3*E*,7*E*)-4,8,12-triméthyltridéca-3,7,11-triènyl]-3,4-dihydrochromèn-6-ol,
le β-tocotriènol ou (2*R*)-2,5,8-triméthyl-2-[(3*E*,7*E*)-4,8,12-triméthyltridéca-3,7,11-triènyl]-3,4-dihydrochromèn-6-ol,
le γ-tocotriènol ou (2*R*)-2,7,8-triméthyl-2-[(3*E*,7*E*)-4,8,12-triméthyltridéca-3,7,11-triènyl]-3,4-dihydrochromèn-6-ol et
le δ-tocotriènol ou (2*R*)-2,8-diméthyl-2-[(3*E*,7*E*)-4,8,12-triméthyltridéca-3,7,11-triènyl]-3,4-dihydrochromèn-6-ol.

Les acétates de tocophérols, comme l'acétate d'α-tocophérol, l'acétate de β-tocophérol, l'acétate de γ-tocophérol ou l'acétate de δ-tocophérol, sont des composés synthétiques obtenus à partir des tocophérols correspondants qui présentent également des propriétés antioxydantes intéressantes. Par conséquent, la vitamine E et l'acétate de vitamine E sont couramment utilisés comme agents antioxydants utilisés pour la préparation de compositions cosmétiques, pharmaceutiques et alimentaires.

La vitamine E est par exemple utilisée comme agent conservateur dans des compositions alimentaires pour éviter le rancissement des aliments. Plus généralement, la vitamine E est associée à des phases grasses insaturées de façon à limiter les phénomènes d'oxydation.

La vitamine E est particulièrement abondante dans le stratum corneum, qui est la première couche de la peau à subir des stress oxydants externes. En effet, au cours de son vieillissement chronologique ou actinique, la peau est soumise à différents stress oxydatifs qui sont soit physiologiques soit résultant de l'action d'agents externes, comme le rayonnement ultra-violet, la pollution atmosphérique ou la consommation de tabac. Ces stress oxydants résultent de l'attaque des cellules de la peau par des espèces radicalaires réactives comme le radical superoxyde, le radical hydroxyle, l'oxygène singulet ou le radical peroxynitrite. Ils provoquent des altérations tissulaires et cellulaires, voire une mortalité cellulaire, et contribuent alors au vieillissement de la peau, qui peut se traduire par une dégradation du collagène, aboutissant à rigidifier la peau. La Vitamine E, du fait de la nature de sa structure chimique, joue un rôle de protection contre le vieillissement de la peau, plus particulièrement provoqué par des agents oxydants radicalaires externes. La Vitamine E joue plus particulièrement un rôle photo-protecteur de la peau contre les rayonnements UV, et notamment contre les dommages aigus de l'inflammation (érythème, coups de soleil) et de l'hyperpigmentation (bronzage) induits par une exposition prolongée aux rayonnements UV ou contre les dommages chroniques du cancer de la peau induit par les rayonnements UV [Burke, K.E. et al., "The effects of topical and oral vitamin E on pigmentation and skin cancer induced by ultraviolet irradiation in Skh:2 hairless mice."; Nutr. Cancer 38:87-97, 2000; Roshchupkin, D. et al., "Inhibition of ultraviolet light-induced erythema byantioxidants.", Arch Dermatol. Res. 266:91-94, 1979; Marenus, K. et al., "The use of antioxidants in providing protection from chronic suberythemal UV-B exposure", 16th IFSCC Conference, 1 Oct. 1990:24-34; Gerrish, K. et al. "Prevention of photocarcinogenesis by dietary vitamin E. " Nutr Cancer 19:125 - 133, 1993.].

L'action de ces stress oxydants sur la peau génère une diminution de la quantité de la Vitamine E dans le stratum corneum, nécessitant donc un régime alimentaire équilibré et/ou un apport en Vitamine E complémentaire au régime alimentaire, sous la forme de l'administration par voie orale, parentérale ou topique de compositions comprenant de la vitamine E.

Le caractère liposoluble de la Vitamine E permet son incorporation aisée dans les phases grasses présentes dans les différentes formes galéniques, comme les émulsions eau-dans-huile ou les émulsions huile-dans-eau et donc dans des formulations cosmétiques et pharmaceutiques administrées par voie topique.

Cependant, des domaines spécifiques d'utilisation de la Vitamine E peuvent nécessiter sa mise en oeuvre dans des milieux aqueux, par exemple :
- Pour la préparation de compositions cosmétiques de nettoyage et/ou de protection et/ou de soin des cheveux et/ou du cuir chevelu ;
- Pour la préparation de gels aqueux et/ou de sérums aqueux destinés à la protection et/ou au soin de la peau et/ou des muqueuses, par exemple des sérums et/ou des gels anti-âge ; ou
- Pour la préparation de compositions alimentaires aqueuses comme les boissons, les boissons lactées, les boissons énergétiques ou les produits laitiers.

Il existe plusieurs manières connus dans l'état de la technique pour résoudre le problème de solubilisation de la Vitamine E dans des phases aqueuses. La demande internationale publiée sous le numéro WO 91/11189 décrit des compositions cosmétiques préparées à partir d'une suspension de vésicules comprenant un sel disodique de phosphate de tocophérol. La demande internationale publiée sous le numéro WO 2003/094882 décrit une composition cosmétique comprenant un sel de sodium de phosphate de tocophérol, se présentant sous la forme d'une solution aqueuse peu concentrée ou d'une dispersion. La demande internationale publiée sous le numéro WO 2005/102267 décrit une composition dont la phase aqueuse comprend le glycinate de tocophérol. Cependant, chacun des éléments de l'état de la technique cité précédemment enseigne que la solubilité du sel de tocophérol dans l'eau, bien qu'améliorée par rapport à celle du tocophérol, n'est cependant pas suffisante pour intégrer des proportions massiques plus importante de Vitamine E dans la forme galénique finale.

Le brevet US 5,990,180 décrit l'utilisation d'un ester de polyglycérol d'un acide gras comportant de 12 à 14 atomes de carbone pour solubiliser la Vitamine E. La demande internationale publiée sous le numéro WO 2008/030312 divulgue l'utilisation de sels d'un mono-ester d'un acide dicarboxylique et d'un alcool gras comportant de 8 à 22 atomes de carbone pour solubiliser la Vitamine E.

La demande de brevet européen publiée sous le numéro EP 0 106 100 A2 divulgue l'utilisation de saponines comme agent de solubilisation de la Vitamine E, et plus particulièrement les saponines provenant d'extrait de plantes, comme la saponine de quillaia, de thé.

La demande de brevet français publiée sous le numéro FR 2 816 517 A1 divulgue la préparation d'alkyl polyglycosides à partir d'huile de fusel et d'au moins un sucre réducteur en milieu catalytique acide, et l'utilisation desdits alkyl polyglycosides sur huile de fusel comme agent solubilisant d'ingrédients huileux ou liposoluble. Les huiles de fusel sont des mélanges d'alcools comprenant notamment des alcools à courtes chaînes comme l'éthanol, le 1-propanol, le 2-propanol, le 1-méthyl-propanol, le 1-butanol, le 2-méthyl butanol et le 3-méthyl butanol, qui nécessitent la mise en oeuvre de mesures de sécurité anti-déflagrantes lors de leur mise en oeuvre industrielle.

La demande internationale publiée sous le numéro WO96/33255A1 décrit des compositions anti-mousses comprenant un alkyl polyglucoside particulier, dont la chaîne alkyle est constituée par le radical 2-éthylhexyle et des agents tensioactifs non-ioniques démoussants choisis parmi ceux comprenant un ou plusieurs groupes choisis parmi les groupes mono-éthoxylés ou poly-éthoxylés, les groupes mono-propoxylés ou poly-propoxylés. Il y est enseigné que les alkyl polyglucosides à chaîne 2-éthyl hexyle sont plus efficaces que les alkyl polygylycosides à chaine hexyle pour solubiliser des tensioactifs non-ioniques démoussants.

La demande internationale publiée sous le numéro WO99/21948A1 divulgue des compositions limpides et stables à hautes concentrations alcalines, dont les propriétés moussantes sont contrôlées, contenant une grande quantité de tensioactifs non-ioniques à base d'oxyde d'alkylène et un hexyl glycoside comme agent hydrotrope. Il y est enseigné que les hexyl glycosides et plus particulièrement le n-hexyl polyglucoside permettent de solubiliser dans des milieux fortement alcalins des tensioactifs non-ioniques.

La demande internationale publiée sous le numéro WO 2012/069730 A1 divulgue des compositions limpides et stables à des concentrations élevées à la fois en espèces alcalines et en espèces électrolytiques, comprenant une grande quantité de tensioactifs non-ioniques à base d'oxyde d'alkylène et le n-heptyl polyglucoside comme agent hydrotrope.

Rien dans l'état de la technique n'enseigne que les alkyl polyglycosides préparés à partir de n-heptanol, ou à partir de 2-éthyl hexanol, et connus pour leur propriétés hydrotropes, à savoir solubiliser dans l'eau des tensioactifs peu solubles dans des milieux fortement alcalins, ou acides, ou électrolytique, puissent constituer des agents solubilisants de la Vitamine E.

C'est pourquoi selon un premier aspect, l'invention a pour objet l'utilisation d'une composition représentée par la formule (I) :

R-O-(G)ₚ-H (I)

dans laquelle R représente un radical choisi parmi les radicaux n-heptyle ou 2-éthyl hexyle, G représente le reste du glucose et p représente un nombre décimal supérieur ou égal à 1,05 et inférieur ou égal à 5, ladite composition de formule (I) consistant en un mélange de composés représentés par les formules (I₁), (I₂), (I₃), (I₄) et (I₅):

R-O-(G)₁-H (I₁)

R-O-(G)₂-H (I₂)

R-O-(G)₃-H (I₃)

R-O-(G)₄-H (I₄)

R-O-(G)₅-H (I₅),

dans les proportions molaires respectives a₁, a₂, a₃, a₄ et a₅, telles que :
- La somme a₁ + a₂ + a₃ + a₄ + a₅ est égale à 1 et que
- chacune des proportions a₁, a₂, a₃, a₄ et a₅ est supérieure ou égale à zéro et inférieure ou égale à un,
comme agent solubilisant dans une composition aqueuse d'au moins un composé (A) choisi parmi l'α-tocophérol, le β-tocophérol, le γ-tocophérol, le δ-tocophérol, l'acétate d'α-tocophérol, l'acétate de β-tocophérol, l'acétate de γ-tocophérol, l'acétate de δ-tocophérol, l'α-tocotriénol, le β-tocotriénol, le γ-tocotriénol et le δ-tocotriénol.

Par α-tocophérol, β-tocophérol, γ-tocophérol, δ-tocophérol, α-tocotriénol, β-tocotriénol, γ-tocotriénol et δ-tocotriénol, on désigne soit les formes diastéréoisomériquement pures de chacun des dits compsés telles qu'ellles sont énoncées dans le préambule de la présente description, soit les racémates desdits composés, par exemple le DL-α-tocophérol.

Dans la formule (I) telle que définie ci-dessus, le groupe R-O est lié à G par le carbone anomérique du reste saccharide, de manière à former une fonction acétal.

Selon un aspect particulier, l'invention a pour objet l'utilisation telle que définie précédemment caractérisée en ce que dans la formule (I), p représente un nombre décimal supérieur ou égal à 1,05 et inférieur ou égal à 2,5.

Selon un autre aspect particulier, l'invention a pour objet l'utilisation telle que définie précédemment pour laquelle, le composé (A) est choisi parmi l'α-tocophérol, le β-tocophérol, le γ-tocophérol, le δ-tocophérol, l'acétate d'α-tocophérol, l'acétate de β-tocophérol, l'acétate de γ-tocophérol et l'acétate de δ-tocophérol et de façon plus particulière le composé (A) est l'α-tocophérol ou l'acétate d'α-tocophérol, par exemple l'utilisation telle que définie précédemment pour laquelle le composé (A) est l'α-tocophérol ou l'utilisation telle que définie précédemment pour laquelle le composé (A) est l'acétate d'α-tocophérol.

Selon un autre aspect particulier, l'invention a pour objet soit l'utilisation telle que définie précédemment pour laquelle dans la formule (I), R représente le radical n-heptyle, G représente le reste du glucose et p représente un nombre décimal supérieur ou égal à 1,05 et inférieur ou égal à 2,5, et le composé (A) est l'α-tocophérol, ou l'acétate d'α-tocophérol ; soit l'utilisation telle que définie précédemment pour laquelle dans la formule (I), R représente le radical 2-éthyl hexyle, G représente le reste du glucose et p représente un nombre décimal supérieur ou égal à 1,05 et inférieur ou égal à 2,5, et le composé (A) est l'α-tocophérol, ou l'acétate d'α-tocophérol.

Selon un autre aspect particulier, l'invention a pour objet l'utilisation telle que définie précédemment caractérisée en ce que le rapport massique entre le composé (A) et la composition représentée par la formule (I), est supérieur ou égal à 1/10 et inférieur ou égal à 6,5/10, plus particulièrement supérieur ou égal à 2/10 et inférieur ou égal à 6,5/10, et encore plus particulièrement supérieur ou égal à 2,5/10 et inférieur ou égal à 6,5/10.

L'invention a aussi pour objet une composition (C₂) comprenant pour 100% de sa masse :
a) de 0,05 % à 12,0 % massique d'au moins une composition représentée par la formule (I) telle que définie précédemment ;
b) de 0,01 à 5,0 % massique d'au moins un composé (A) choisi parmi l'α-tocophérol, le β-tocophérol, le γ-tocophérol, le δ-tocophérol, l'acétate d'α-tocophérol, l'acétate de β-tocophérol, l'acétate de γ-tocophérol, l'acétate de δ-tocophérol, l'α-tocotriénol, le β-tocotriénol, le γ-tocotriénol ou le δ-tocotriénol, et
c) de 83,0 % à 99,94 % massique d'eau.

Selon un aspect particulier, l'invention a pour objet une composition (C₂) telle que définie précédemment pour laquelle, dans la formule (I), p représente un nombre décimal supérieur ou égal à 1,05 et inférieur ou égal à 2,5.

Selon un autre aspect particulier, l'invention a pour objet une composition (C₂) telle que définie précédemment dans laquelle, le composé (A) est choisi parmi l'α-tocophérol, le β-tocophérol, le γ-tocophérol, le δ-tocophérol, l'acétate d'α-tocophérol, l'acétate de β-tocophérol, l'acétate de γ-tocophérol ou l'acétate de δ-tocophérol; et plus particulièrement une composition (C₂) telle que définie précédemment, caractérisée en ce que le composé (A) est l'α-tocophérol ou l'acétate d'α-tocophérol ; par exemple une composition (C₂) telle que définie précédemment, dans laquelle le composé (A) est l'α-tocophérol ou une composition (C₂) telle que définie précédemment dans laquelle le composé (A) est l'acétate d'α-tocophérol.

Selon un autre aspect particulier, l'invention a pour objet une composition (C₂) telle que définie précédemment pour laquelle dans la formule (I) R représente le radical n-heptyle, G représente le reste pour laquelle dans la formule (I), R représente le radical n-heptyle, G représente le reste du glucose et p représente un nombre décimal supérieur ou égal à 1,05 et inférieur ou égal à 2,5 et dans laquelle le composé (A) est l'α-tocophérol, ou l'acétate d'α-tocophérol ; ou bien une composition (C₂) telle que définie précédemment pour laquelle dans la formule (I), R représente le radical 2-éthyl hexyle, G représente le reste du glucose et p représente un nombre décimal supérieur ou égal à 1,05 et inférieur ou égal à 2,5, et dans laquelle le composé (A) est l'α-tocophérol, ou l'acétate d'α-tocophérol.

Selon un autre aspect particulier, l'invention a pour objet une composition (C₂) telle que définie précédemment dans laquelle le rapport massique entre le composé (A) et la composition représentée par la formule (I), est supérieur ou égal à 1/10 et inférieur ou égal à 6,5/10, plus particulièrement supérieur ou égal à 2/10 et inférieur ou égal à 6,5/10 et encore plus particulièrement supérieur ou égal à 2,5/10 et inférieur ou égal à 6,5/10.

La composition (C₂) selon l'invention est préparée par mélange de ses constituants, sous agitation mécanique, à une vitesse d'agitation comprise entre 50 tours/minute et 500 tours/minutes, plus particulièrement entre 50 tours/minutes et 100 tours/minutes, à une température comprise entre 20°C et 80°C, plus particulièrement entre 20°C et 60°C, et encore plus particulièrement entre 20°C et 45°C.

L'invention a aussi pour objet l'utilisation de la composition (C₂) telle que définie ci-dessus pour prévenir les effets inesthétiques du vieillissement de la peau, des cheveux, du cuir chevelu ou des muqueuses et plus particulièrement ceux induits par des dommages oxydatifs provenant de radicaux libres, notamment ceux générés par les rayonnements du soleil, comme les rayonnements ultra-violets du soleil.

Par « vieillissement de la peau », on entend un phénomène physiologique évolutif qui se manifeste par un teint jaunâtre ou translucide lié à l'amincissement du derme, la fonte du pannicule adipeux, les rides et/ou la réduction de la tonicité cutanée.

Par « vieillissement des cheveux», on entend un phénomène physiologique évolutif qui se manifeste par l'endommagement des cuticules et aboutissant à un état de surface des cheveux moins lisse, à l'endommagement des fibres, pouvant alors provoquer des cassures et/ou à une perte de brillance des cheveux.

L'invention a aussi pour objet l'utilisation de la composition (C₂) telle que définie ci-dessus pour la préparation d'une composition destinée au nettoyage de la peau, des cheveux, du cuir chevelu ou des muqueuses.

L'expression « nettoyage de la peau, des cheveux, du cuir chevelu ou des muqueuses» désigne toute action destinée à permettre l'élimination de salissures présentes sur la peau, les cheveux, le cuir chevelu ou les muqueuses des humains ou des animaux. Comme exemples de salissures présentes sur la peau, les cheveux, le cuir chevelu ou les muqueuses des humains ou des animaux, il y a les poussières, la terre, les sécrétions sébacées, la sueur, les pellicules, les cellules mortes, des microorganismes ou diverses substances chimiques comme les résidus de compositions de maquillage et de soins de la peau, des cheveux, du cuir chevelu ou des muqueuses.

Lorsqu'elle est utilisée à des fins cosmétiques, à savoir modifier l'apparence externe de la peau, des cheveux, du cuir chevelu ou des muqueuses, par exemple pour prévenir des effets inesthétiques du vieillissement, ou pour nettoyer, la composition (C₂) objet de la présente invention est plus particulièrement administrée par voie topique.

L'expression "topique" signifie que la composition (C₂) selon l'invention est mise en oeuvre par application sur la peau, les cheveux, le cuir chevelu ou les muqueuses, qu'il s'agisse d'une application directe ou d'une application indirecte, lorsque par exemple la composition (C₂) selon l'invention est incorporée dans un support destiné à être mis en contact avec la peau (papier, lingette, textile, dispositif transdermique, etc.).

La composition topique (C₂) selon l'invention peut être conditionnée dans un flacon, et également sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on »).

La composition topique (C₂) selon l'invention comporte généralement des additifs chimiques habituellement mis en oeuvre dans le domaine des formulations à usage topique, en particulier cosmétiques, dermocosmétiques, pharmaceutiques ou dermopharmaceutiques, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les agents plastifiants, les agents émulsionnants et co-émulsionnants, les agents opacifiants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les repellents pour les insectes.

Comme exemples de tensioactifs moussants et/ou détergents, éventuellement présents dans la composition topique (C₂) selon l'invention, on peut citer les tensioactifs moussants et/ou détergents anioniques, cationiques, amphotères ou non ioniques.

Parmi les tensioactifs anioniques moussants et/ou détergents que l'on peut inclure dans la composition topique (C₂) selon l'invention, on peut citer les sels de métaux alcalins, les sels de métaux alcalino-terreux, les sels d'ammonium, les sels d'amines, les sels d'aminoalcools d'alkyléthers sulfates, d'alkylsulfates, d'alkylamidoéthersulfates, d'alkylarylpolyéthersulfates, de monoglycérides sulfates, d'alpha-oléfinesulfonates, de paraffines sulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents que l'on peut inclure dans la composition topique (C₂) selon l'invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents que l'on peut inclure dans la composition topique (C₂) selon l'invention, on peut citer particulièrement les dérivés d'ammoniums quaternaires.

Parmi les tensioactifs non ioniques moussants et/ou détergents que l'on peut inclure dans la composition topique (C₂) selon l'invention, on peut citer plus particulièrement les alkyl polyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants, éventuellement présents dans la composition topique (C₂) selon l'invention, on peut citer les esters gras d'alkyl polyglycosides éventuellement alcoxylés, comme les esters de méthyl polyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses par exemple le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants éventuellement présents dans la composition topique (C₂) selon l'invention, on peut citer les polymères de type polyélectrolytes, linéaires ou branchés ou réticulés, par exemple, l'homopolymère de l'acide acrylique, l'homopolymère de l'acide méthacrylique, l'homopolymère de l'acide-2-méthyl-[(1-oxo-2-propényl)amino] 1-propanesulfonique (AMPS), les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthylacrylamide, les copolymères de l'AMPS et du tris(hydroxy-méthyl) acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthylacrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone. Les polymères, et plus particulièrement les copolymères, les terpolymères et les tétrapolymères réticulés comprenant de l'AMPS et des macromères, comme ceux décrits dans les demandes de brevet européen publiées sous les numéros EP 1 069 142 A1 et EP 1 339 789 A2, dans la demande internationale publiée sous le numéro WO 2011/030044 A1 et dans la demande de brevet de brevet français publiée sous le numéro 2910899A1.

Les polymères de type polyélectrolytes, linéaires ou branchés ou réticulés éventuellement présents dans la composition topique (C₂) selon l'invention peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés éventuellement présents dans la composition topique selon l'invention peuvent être sélectionnés parmi les produits commercialisés sous les appellations SIMULGEL™EG, SIMULGEL™EPG, SEPIGEL™305, SIMULGEL™600, SIMULGEL™NS, SIMULGEL™INS100, SIMULGEL™FL, SIMULGEL™A, SIMULGEL™SMS88, SEPINOV™EMT10, SEPIPLUS™400, SEPIPLUS™265, SEPIPLUS™S, SEPIMAX™ZEN, ARISTOFLEX™HMB, ARISTOFLEX™VELVET, ARISTOFLEX™AVC, ARISTOFLEX™AVS, NOVEMER™EC-1, NOVEMER™EC-2, FLOCARE™ET25, FLOCARE™ET75, FLOCARE™ET26, FLOCARE™ET30, FLOCARE™ET58, FLOCARE™PSD30, VISCOLAM™AT64, VISCOLAM™AT100.

Comme exemples d'agents épaississants et/ou gélifiants éventuellement présents dans la composition topique (C₂) selon l'invention, on peut citer les polysaccharides constitués uniquement d'oses, par exemple les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, par exemple les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Comme exemples d'agents épaississants et/ou gélifiants éventuellement présents dans la composition topique (C₂) selon l'invention, on peut citer les polysaccharides constitués de dérivés d'oses, par exemple les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants éventuellement présents dans la composition topique (C₂) selon l'invention, on peut citer la cellulose, les dérivés de cellulose par exemple la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants éventuellement présents dans la composition topique (C₂) selon l'invention, on peut citer par exemple les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants éventuellement présents dans la composition topique (C₂) selon l'invention, on peut citer l'eau, les solvants organiques par exemple le glycérol, le diglycérol, les oligomères du glycérol, l'éthylène glycol, le propylène glycol, le butylèneglycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples de tensioactifs émulsionnants éventuellement présents dans la composition topique (C₂) selon l'invention, on peut citer les tensioactifs non ioniques, des tensioactifs anioniques, des tensioactifs cationiques.

Comme exemples de tensioactifs non-ioniques émulsionnants éventuellement présents dans la composition topique (C₂) selon l'invention, on peut citer les esters d'acides gras et de sorbitol, par exemple les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre 5 moles et 150 moles d'oxyde d'éthylène, par exemple la composition comprenant de l'acide stéarique éthoxylé à 135 moles d'oxyde d'éthylène et du stéarate de glycérol commercialisée sous l'appellation SIMULSOL™165 ; les esters de mannitan ; les esters de mannitan éthoxylés ; les esters de sucrose ; les esters de méthyl glucoside ; les alkyl polyglycosides comportant un radical aliphatique, linéaire ou ramifié, saturé ou insaturé, et comportant de 14 à 36 atomes de carbone, par exemple le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyl dodécyl polyxyloside, le (12-hydroxy stéaryl) polyglucoside ; les compositions d'alcools gras linéaires ou ramifiés, saturés ou insaturés, et comportant de 14 à 36 atomes de carbone, et d' alkyl polyglycosides tels que décrits précédemment.

Comme exemples de tensioactifs anioniques éventuellement présents dans la composition topique (C₂) selon l'invention, on peut citer le glycéryl stéarate citrate, le cétéarylsulfate, les savons par exemple le stéarate de sodium ou le stéarate de triéthanolammonium, les dérivés N-acylés d'acides aminés salifiés par exemple le stéaroyl glutamate.

Comme exemples de tensioactifs cationiques émulsionnants éventuellement présents dans la composition topique (C₂) selon l'invention, on peut citer les aminoxydes, le quaternium-82 et les tensioactifs décrits dans la demande internationale publiée sous le numéro WO 96/00719 et principalement ceux dont la chaîne grasse comprend au moins 16 atomes de carbone.

Comme exemples d'agents opacifiants et/ou nacrants éventuellement présents dans la composition topique (C₂) selon l'invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans la composition topique (C₂) selon l'invention, on peut citer des dérivés N-acylés d'acides aminés, par exemple la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica.

Comme exemples d'agents déodorants éventuellement présents dans la composition topique (C₂) selon l'invention, on peut citer par exemple les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3-propanediol ; l'acide salicylique ; le bicarbonate de sodium; les cyclodextrines ; les zéolithes métalliques ; le Triclosan™; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles éventuellement présentes dans la composition topique (C₂) selon l'invention, on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires éventuellement présentes dans la composition topique (C₂) selon l'invention, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs éventuellement présents dans la composition topique (C₂) selon l'invention, on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique ( par exemple l'ascorbyl glucoside) et ses esters (par exemple l'acétate de tocophérol), les vitamines B3 ou B10 (niacinamide et ses dérivés) ; les composés montrant une action éclaircissante ou dépigmentante de la peau par exemple le ω-undecylènoyl phénylalanine commercialisé sous l'appellation SEPIWHITE™MSH, le SEPICALM™VG, le mono ester et/ou le diester de glycérol du ω-undecylènoyl phénylalanine, les ω-undecylènoyl dipeptides, l'arbutine, l'acide kojique, l'hydroquinone ; les composés montrant une action apaisante notamment le SEPICALM™S, l'allantoïne et le bisabolol ; les agents antiinflammatoires ; les composés montrant une action hydratante par exemple l'urée, les hydroxyurées, le glycérol, les polyglycérols, le glycérolglucoside, le diglycérolglucoside, les polyglycérylglucosides, le xylitylglucoside ; les extraits végétaux riches en polyphénols par exemple les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés par exemple le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, par exemple le LIPACIDE™C8G, le LIPACIDE™UG, le SEPICONTROL™A5 ; l'OCTOPIROX™ ou le SENSIVA™SC50 ; les composés montrant une propriété énergisante ou stimulante comme le Physiogényl™, le panthénol et ses dérivés comme le SEPICAP™MP ; les actifs anti-âge comme le SEPILIFT™DPHP, le LIPACIDE™PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermoépidermique ; les actifs augmentant la synthèse des composants de la matrice extracellulaire par exemple le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ; les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de *centalla asiatica,* de fucus, de romarin, de saule ; les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose. Comme exemples de filtres solaires éventuellement présents dans la composition topique (C₂) selon l'invention, on peut citer tous ceux figurant dans la directive cosmétique 76/768/CEE modifiée annexe VII.

Parmi les filtres organiques solaires éventuellement présents dans la composition topique (C₂) selon l'invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'(éthyl hexyle), le 4-isopropyl cinnamate d'éthyle, le 2,5-diisopropyl cinnamate de méthyle, le 4-méthoxy cinnamate de propyle, le 4-méthoxy cinnamate d'isopropyle, le 4-méthoxy cinnamate d'isoamyle, le 4-méthoxy cinnamate d'octyle, le 4-méthoxy cinnamate de (2-éthyl hexyle), le 4-méthoxy cinnamate de (2-éthoxy éthyle), le 4-méthoxy cinnamate de cyclohexyle, le cinnamate d'éthyl-α-cyano-β-phényle, le cinnamate de (2-éthyl hexyle), -□α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, l'ester 4,4-((6-(((1,1-diméthyléthyl) amino) carbonyl) phenyl) amino)-1,3,5-triazine-2,4-diyl diimino) bis-(2-éthylhexyl) de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", éventuellement présents dans la composition topique (C₂) selon l'invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

La composition topique (C₂) selon l'invention peut être plus particulièrement une suspension de particules solides. Lesdites particules solides suspendues présentes dans la composition topique (C₂) selon l'invention peuvent revêtir différentes géométries, régulières ou irrégulières, et se présenter sous forme de perles, de billes, de tiges, de paillettes, de lamelles ou de polyèdres. Ces particules solides se caractérisent par un diamètre moyen apparent compris entre 1 micromètre et 5 millimètres, plus particulièrement entre 10 micromètres et 1 millimètre. Parmi les particules solides, on peut citer les micas, l'oxyde de fer, l'oxyde de titane, l'oxyde de zinc, l'oxyde d'aluminium, le talc, la silice, le kaolin, les argiles, le nitrure de bore, le carbonate de calcium, le carbonate de magnésium, l'hydrogénocarbonate de magnésium, les pigments colorés inorganiques, les polyamides comme le nylon-6, les polyéthylènes, les polypropylènes, les polystyrènes, les polyesters, les polymères acryliques ou méthacryliques comme les polyméthylméthacrylates, le polytétrafluoroéthylène, les cires cristallines ou microcristallines, des sphères poreuses, le sulphure de sélénium, le pyrithione de zinc, les amidons, les alginates, les fibres de végétaux, les particules de Loofah, les particules d'éponges.

Selon un autre aspect, l'invention a pour objet une composition (C₂) telle que définie ci-dessus pour la mise en oeuvre d'une méthode de traitement thérapeutique du corps humain ou animal destinée à traiter et/ou prévenir les rougeurs et/ou les irritations visibles de la peau et/ou du cuir chevelu dues aux rayonnements du soleil.

La composition (C₂) telle que définie ci-dessus peut par exemple être administrée par voie topique.

L'étude expérimentale suivante illustre l'invention sans toutefois la limiter.

### 1) Préparation de compositions représentées par la formule (I) et évaluation de leurs propriétés solubilisantes selon l'invention.

### 1.1 Préparation d'une composition de n-heptyl polyglucosides

On introduit 2,7 équivalents molaires de n-heptanol dans un réacteur en verre à double enveloppe, dans laquelle circule un fluide caloporteur, et muni d'une agitation efficace, à une température de 40°C. Un équivalent molaire de glucose anhydre est alors ajouté progressivement au milieu réactionnel pour permettre sa dispersion homogène, puis 0,15% massique d'acide sulfurique à 98% et 0,15% massique d'acide hypophosphoreux à 50% pour 100% de la masse constituée par la somme de la massse du glucose et de la masse du n-heptanol sont introduits dans la dispersion homogène préalablement préparée. Le milieu réactionnel est placé sous un vide partiel d'environ 180 mbars, et maintenu à une température de 100°C-105°C pendant une durée de 4 heures avec évacuation de l'eau formée au moyen d'un montage de distillation. Le milieu réactionnel est ensuite refroidi à 85°C-90°C et neutralisé par ajout de soude à 40%, pour amener le pH d'une solution à 5% de ce mélange à une valeur d'environ 7,0. La milieu réactionnel ainsi obtenu est ensuite vidangée à une température de 70°C et filtré pour éliminer les grains de glucose n'ayant pas réagi. Le filtrat est ensuite introduit dans un réacteur en verre à double enveloppe, dans laquelle circule un fluide caloporteur, muni d'une agitation efficace et d'un dispositif de distillation. L'excès d'heptanol est ensuite éliminé par distillation à une température de 120°C sous vide partiel compris entre environ 100 mbars et 50 mbars. Le milieu réactionnel ainsi distillé est aussitôt dilué par l'ajout d'une quantité d'eau de façon à atteindre une concentration en milieu réactionnel d'environ 60%. Après homogénéisation pendant 30 minutes à une température de 50°C, la composition (X₁) obtenue est vidangée.

Les caractéristiques analytiques de la composition (X₁) ainsi obtenue comprenant des n-heptyl polyglucosides sont rassemblées dans le tableau 1 ci-dessous.

**Tableau 1 : Caractéristiques analytiques de la composition (X₁).**

| | Composition (X₁) |
|---|---|
| Aspect à 20°C (Détermination visuelle) | Liquide |
| Indice d'acide (Norme NFT 60204) | 1,7 |
| Indice d'hydroxyle sur extrait sec (Norme USP XXI NF XVI 01/01/1995) | 813,9 |
| Eau (% massique) (Norme NFT 73201) | 58,8 % |
| Teneur résiduelle en n-heptanol (chromatographie en phase gazeuse) en % massique | 0,22 % |

### 1.2 Evaluation des propriétés solubilisantes de la vitamine E de n-heptyl polyglucosides, de 2-éthylhexyl polyglucosides et de n-butyl polyglucosides dans l'eau.

Les propriétés solubilisantes de la composition (X₁) de n-heptyl polyglucosides, obtenue selon le procédé précédemment décrit, de la composition de (2-éthyl hexyl) polyglucosides, commercialisée sous le nom de marque AG 6202 par la société NOBEL AKZO (composition (X₂)), et de la composition de n-butyl polyglucosides, commercialisée sous le nom de marque SIMULSOL™SL4 (composition (X₄)), ont été évaluées comparativement à celles d'une composition d'huile de ricin hydrogénée et éthoxylée à 40 moles d'oxyde d'éthylène (composition (X₃)), dont le nom INCI est « PEG-40 hydrogenated castor oil » et dont le numéro CAS est 61788-85-0, commercialisée sous l'appellation SIMULSOL™1293 par la société SEPPIC, représentant l'état de la technique, selon la méthode d'évaluation décrite ci-dessous pour le DL α-Tocophérol.

### 1.2.1- Principe de la méthode d'évaluation de la solubilisation en milieu aqueux du DL α-Tocophérol par les compositions selon l'invention et par la composition selon l'état de la technique.

Cette méthode a pour objet de déterminer le pouvoir solubilisant de compositions tensioactives, (X₁) et (X₂), en milieu aqueux pour le DL α-Tocophérol insoluble en milieu aqueux par rapport à des compositions tensioactives de l'état de la technique (X₃) et (X₄).

### 1.2.2- Protocole expérimental.

Pour chaque composition (X₁), (X₂), (X₃) et (X₄) à évaluer, on introduit dans un flacon de verre de 150 cm³ une quantité de 10 grammes en matière sèche de ladite composition à évaluer. On introduit ensuite une fraction massique déterminée de DL α-Tocophérol à solubiliser. Une quantité d'eau distillée est ajoutée de manière complémentaire de façon à obtenir une masse de 100 grammes. La température est régulée à 20°C ou 45°C. Un barreau magnétique aimanté est introduit dans le flacon de verre qui est ensuite placé sous agitation magnétique à une vitesse de 80 tours/min pendant une durée de 60 minutes à une température de 20°C ou de 45°C. A l'issue de cette durée, on note l'aspect visuel obtenu. Si le mélange est limpide, un autre essai est mené dans les conditions opératoires décrites ci-dessus avec une fraction massique de DL α-Tocophérol plus élevée ; si le mélange est trouble et non limpide, un autre essai est mené dans les conditions opératoires décrites ci-dessus avec une fraction massique de DL α-Tocophérol moins élevée.

### 1.2.3- Expression des résultats

L'aspect visuel de la solution obtenue selon le protocole de la section 1.2.2 de la présente demande est noté par l'expérimentateur et qualifié de « limpide » ou de « trouble » selon les cas.

### 1.2.4- Caractérisation de la solubilisation en milieu aqueux, à une température de 20°C du DL α-Tocophérol par les compositions (X₁) et (X₂) comprenant les composés de formule (I) selon l'invention et par les compositions (X₃) et (X₄) de l'état de la technique.

Le protocole expérimental décrit dans la section 1.2.2 de la présente demande a été mis en oeuvre à une température de 20°C pour les compositions (X₁) et (X₂) selon l'invention et pour les compositions (X₃) et (X₄) de l'état de la technique.

Les aspects des solutions préparées par la mise en oeuvre du protocole opératoire pour chacune des compositions (X₁), (X₂), (X₃) et (X₄) utilisées pour solubiliser le DL α-Tocophérol à 20°C ont été relevés par l'expérimentateur et consignés respectivement dans les tableaux 2, 3, 4 et 5 ci-dessous.

**Tableau 2 : Aspect de solutions comprenant la composition tensioactive non-ionique (X₁) et une quantité variable de DL α-Tocophérol à 20°C**

| Fraction massique de DL α-Tocophérol | Aspect des solutions |
|---|---|
| 0% | Limpide |
| 0,1% | Limpide |
| 0,5% | Limpide |
| 1,0% | Limpide |
| 2,0% | Limpide |
| 4,0% | Limpide |
| 5,0% | Limpide |
| 6,0% | Limpide |
| 6,5% | Limpide |
| 6,7% | Trouble |
| 7,0% | Trouble |

**Tableau 3 : Aspect de solutions comprenant la composition tensioactive non-ionique (X₂) et une quantité variable de DL α-Tocophérol à 20°C.**

| Fraction massique de DL α-Tocophérol | Aspect des solutions |
|---|---|
| 0% | Limpide |
| 0,1 % | Limpide |
| 0,5 % | Limpide |
| 1,0 % | Limpide |
| 2,0 % | Limpide |
| 4,0 % | Limpide |
| 4,1 % | Limpide |
| 4,2 % | Trouble |
| 4,5 % | Trouble |
| 5,0 % | Trouble |

**Tableau 4 : Aspect de solutions comprenant la composition tensioactive non-ionique (X₃) et une quantité variable de DL α-Tocophérol à 20°C.**

| Fraction massique de DL α-Tocophérol | Aspect des solutions |
|---|---|
| 0 % | Limpide |
| 0,1 % | Trouble |
| 0,5 % | Trouble |
| 1,0 % | Trouble |

**Tableau 5 : Aspect de solutions comprenant la composition tensioactive non-ionique (X₄) et une quantité variable de DL α-Tocophérol à 20°C.**

| Fraction massique de DL α-Tocophérol | Aspect des solutions |
|---|---|
| 0 % | Limpide |
| 0,5 % | Trouble |
| 1,5 % | Trouble |
| 3,0 % | Trouble |
| 5,0 % | Trouble |
| 6,5 % | Trouble |

### 1.2.5- Caractérisation de la solubilisation en milieu aqueux, à une température de 45°C du DL α-Tocophérol par les compositions (X₁) et (X₂) comprenant les composés de formule (I) selon l'invention et par la composition (X₃) de l'état de la technique.

Le protocole expérimental décrit dans la section 1.2.2 de la présente demande a été mis en oeuvre à une température de 45°C pour les compositions (X₁) et (X₂) selon l'invention et pour la composition (X₃) de l'état de la technique.

Les aspects des solutions préparées par la mise en oeuvre du protocole opératoire pour chacune des compositions (X₁), (X₂) et (X₃) utilisées pour solubiliser le DL α-Tocophérol à 45°C ont été relevés par l'expérimentateur et consignés respectivement dans les tableaux 6, 7 et 8 ci-dessous.

**Tableau 6 : Aspect de solutions comprenant la composition tensioactive non-ionique (X₁) et une quantité variable de DL α-Tocophérol à 45°C.**

| Fraction massique de DL α-Tocophérol | Aspect des solutions |
|---|---|
| 0% | Limpide |
| 1,0 % | Limpide |
| 2,0 % | Limpide |
| 4,0 % | Limpide |
| 5,0 % | Limpide |
| 6,0 % | Limpide |
| 6,5% | Limpide |
| 6,7 % | Trouble |
| 7,0 % | Trouble |

**Tableau 7 : Aspect de solutions comprenant la composition tensioactive non-ionique (X₂) et une quantité variable de DL α-Tocophérol à 45°C**

| Fraction massique de DL α-Tocophérol | Aspect des solutions |
|---|---|
| 0% | Limpide |
| 1,0 % | Limpide |
| 2,0 % | Limpide |
| 4,0 % | Limpide |
| 4,1 % | Limpide |
| 4,2 % | Trouble |
| 4,5 % | Trouble |
| 5,0 % | Trouble |

**Tableau 8 : Aspect de solutions comprenant la composition tensioactive non-ionique (X₃) et une quantité variable de DL α-Tocophérol à 45°C**

| Fraction massique de DL α-Tocophérol | Aspect des solutions |
|---|---|
| 0% | Limpide |
| 0,1 % | Limpide |
| 0,5 % | Trouble |
| 1,0 % | Trouble |

### 1.2.6 Analyse des résultats.

Les compositions (X₁) et (X₂), comprenant respectivement des n-heptyl polyglucosides et des (2-éthyl hexyl) polyglucosides, permettent de solubiliser dans l'eau des proportions massiques respectives de 6,5% et de 4,1% de DL α-Tocophérol à une température de 20°C alors que dans les mêmes conditions opératoires les compositions (X₃) et (X₄) comparatives ne permettent pas de solubiliser le DL α-Tocophérol dans l'eau.

Les compositions (X₁) et (X₂), comprenant respectivement des n-heptyl polyglucosides et des (2-éthyl hexyl) polyglucosides se caractérisent par les mêmes capacités de solubilisation du DL α-Tocophérol dans l'eau à une température opératoire de 45°C.

### 1.2.7 Stabilité des solutions de DL α-Tocophérol en présence des compositions (X₁) et (X₂).

a) Deux solutions comprenant chacune 10% massique en matière sèche de la composition (X₁) et des teneurs massiques en DL α-Tocophérol respectivement de 6,0% et de 6,5%, ont été conservées pendant 7 jours sans agitation, dans une enceinte climatisée et régulée à une température de 20°C. A l'issue de cette durée, chacune des solutions présente un aspect limpide.
b) Deux solutions comprenant chacune 10% massique en matière sèche de la composition (X₂) et des teneurs massiques en DL α-Tocophérol respectivement de 3,0% et de 4,0%, ont été conservées pendant 7 jours sans agitation, dans une enceinte climatisée et régulée à une température de 20°C. A l'issue de cette durée, chacune des solutions présente un aspect limpide.
c) Deux solutions comprenant chacune 10% massique en matière sèche de la composition (X₁) et des teneurs massiques en DL α-Tocophérol respectivement de 6,0% et de 6,5%, ont été conservées pendant 7 jours sans agitation, dans une enceinte climatisée et régulée à une température de 45°C. A l'issue de cette durée, chacune des solutions présente un aspect limpide.
d) Deux solutions comprenant chacune 10% massique en matière sèche de la composition (X₂) et des teneurs massiques en DL α-Tocophérol respectivement de 3,0% et de 4,0%, ont été conservées pendant 7 jours sans agitation, dans une enceinte climatisée et régulée à une température de 45°C. A l'issue de cette durée, chacune des solutions présente un aspect limpide.

### 1.2.7 Conclusions

Les compositions (X₁) et (X₂) comprenant resepectivement des n-heptyl polyglucosides et des (2-éthyl hexyl) polyglucosides permettent de solubiliser de façon stable le DL α-Tocophérol par rapport aux agents solubilisants connus dans l'état de la technique.

## Revendications

1. Utilisation d'une composition représentée par la formule (I) :
R-O-(G)ₚ-H (I)
dans laquelle R représente un radical choisi parmi les radicaux n-heptyle ou 2-éthyl hexyle, G représente le reste du glucose et p représente un nombre décimal supérieur ou égal à 1,05 et inférieur ou égal à 5, ladite composition de formule (I) consistant en un mélange de composés représentés par les formules (I₁), (I₂), (I₃), (I₄) et (I₅) :
R-O-(G)₁-H (I₁)
R-O-(G)₂-H (I₂)
R-O-(G)₃-H (I₃)
R-O-(G)₄-H (I₄)
R-O-(G)₅-H (I₅)
dans les proportions molaires respectives a₁, a₂, a₃, a₄ et a₅, telles que :
- La somme a₁ + a₂ + a₃ + a₄ + a₅ est égale à 1 et que
- chacune des porportions a₁, a₂, a₃, a₄ et a₅ est supérieure ou égale à zéro et inférieure ou égale à un,
comme agent solubilisant dans une composition aqueuse d'au moins un composé (A) choisi parmi l'α-tocophérol, le β-tocophérol, le γ-tocophérol, le δ-tocophérol, l'acétate d'α-tocophérol, l'acétate de β-tocophérol, l'acétate de γ-tocophérol, l'acétate de δ-tocophérol, l'α-tocotriénol, le β-tocotriénol, le γ-tocotriénol et le δ-tocotriénol.

2. Utilisation telle que définie à la revendication 1, **caractérisée en ce que** dans la formule (I), p représente un nombre décimal supérieur ou égal à 1,05 et inférieur ou égal à 2,5.

3. Utilisation telle que définie à l'une ou quelconque des revendications 1 ou 2, **caractérisée en ce que** le composé (A) est l'α-tocophérol ou l'acétate d'α-tocophérol.

4. Utilisation telle que définie à l'une ou quelconque des revendications 1 à 3, **caractérisée en ce que** le rapport massique entre le composé (A) et la composition représentée par la formule (I), est supérieur ou égal à 1/10 et inférieur ou égal à 6,5/10.

5. Composition (C₂) comprenant pour 100% de sa masse :
a) de 0,05 % à 12,0 % massique d'au moins une composition représentée par la formule (I) telle que définie à la revendication 1 ;
b) de 0,01 à 5,0 % massique d'au moins un composé (A) choisi parmi l'α-tocophérol, le β-tocophérol, le γ-tocophérol, le δ-tocophérol, l'acétate d'α-tocophérol, l'acétate de β-tocophérol, l'acétate de γ-tocophérol, l'acétate de δ-tocophérol, l'α-tocotriénol, le β-tocotriénol, le γ-tocotriénol et le δ-tocotriénol.
c) de 83,0 % à 99,94 % massique d'eau.

6. Composition (C₂) telle que définie à la revendication 5, **caractérisée en ce que** le composé (A) est l'α-tocophérol ou l'acétate d'α-tocophérol.

7. Composition (C₂) telle que définie à l'une des revendications 5 ou 6, **caractérisée en ce que** le rapport massique entre le composé (A) et la composition représentée par la formule (I), est supérieur ou égal à 1/10 et inférieur ou égal à 6,5/10.

8. Utilisation non-thérapeutique de la composition (C₂) telle que définie à l'une quelconque des revendications 5 à 7, pour prévenir les effets inesthétiques du vieillissement de la peau, des cheveux, du cuir chevelu ou des muqueuses.

9. Utilisation de la composition (C₂) telle que définie à l'une des revendications 5 à 7 pour la préparation d'une composition destinée au nettoyage de la peau, des cheveux, du cuir chevelu ou des muqueuses.

10. Composition (C₂) telle que définie à l'une des revendications 5 à 7 pour la mise en oeuvre d'une méthode de traitement thérapeutique du corps humain ou animal destinée à traiter et/ou prévenir les rougeurs et/ou les irritations visibles de la peau et/ou du cuir chevelu dues aux rayonnements du soleil.

## Patentansprüche

1. Verwendung einer Zusammensetzung, dargestellt durch die Formel (I):
R-O-(G)ₚ-H (I)
worin R einen Rest darstellt, der aus den n-Heptyl- oder 2-Ethylhexylresten ausgewählt ist, G den Glucose-Rest darstellt und p eine Dezimalzahl größer oder gleich 1,05 und kleiner oder gleich 5 darstellt, wobei die Zusammensetzung der Formel (I) aus einem Gemisch von Verbindungen besteht, die durch die Formeln (I₁), (I₂), (I₃), (I₄) und (I₅) dargestellt sind:
R-O-(G)₁-H (I₁)
R-O-(G)₂-H (I₂)
R-O-(G)₃-H (I₃)
R-O-(G)₄-H (I₄)
R-O-(G)₅-H (I₅)
in den jeweiligen Molverhältnissen a₁ a₂, a₃, a₄ und a₅, derart dass:
- die Summe a₁ + a₂ + a₃ + a₄ + a₅ gleich 1 ist, und dass
- jedes der Verhältnisse a₁, a₂, a₃, a₄ und a₅ größer oder gleich Null und kleiner oder gleich Eins ist,
als Lösungsvermittler in einer wässrigen Zusammensetzung aus mindestens einer Verbindung (A), ausgewählt aus α-Tocopherol, β-Tocopherol, γ-Tocopherol, 6-Tocopherol, α-Tocopherolacetat, β-Tocopherolacetat, γ-Tocopherolacetat, 6-Tocopherolacetat, α-Tocotrienol, β-Tocotrienol, γ-Tocotrienol und δ-Tocotrienol.

2. Verwendung, wie in Anspruch 1 definiert, **dadurch gekennzeichnet, dass** in Formel (I) p eine Dezimalzahl größer oder gleich 1,05 und kleiner oder gleich 2,5 darstellt.

3. Verwendung, wie in einem der Ansprüche 1 oder 2 definiert, **dadurch gekennzeichnet, dass** die Verbindung (A) α-Tocopherol oder α-Tocopherolacetat ist.

4. Verwendung, wie in einem der Ansprüche 1 bis 3 definiert, **dadurch gekennzeichnet, dass** das Massenverhältnis zwischen der Verbindung (A) und der durch die Formel (I) dargestellten Zusammensetzung größer oder gleich 1/10 und kleiner oder gleich 6,5/10 ist.

5. Zusammensetzung (C₂), die für 100 % ihrer Masse Folgendes umfasst:
a) von 0,05 bis 12,0 Masse-% von mindestens einer Zusammensetzung, dargestellt durch die Formel (I), wie in Anspruch 1 definiert;
b) von 0,01 bis 5,0 Masse-% von mindestens einer Verbindung (A), ausgewählt aus α-Tocopherol, β-Tocopherol, γ-Tocopherol, δ-Tocopherol, α-Tocopherolacetat, β-Tocopherolacetat, γ-Tocopherolacetat, δ-Tocopherolacetat, α-Tocotrienol, β-Tocotrienol, γ-Tocotrienol und δ-Tocotrienol.
c) von 83,0 bis 99,94 Masse-% Wasser.

6. Zusammensetzung (C₂), wie in Anspruch 5 definiert, **dadurch gekennzeichnet, dass** die Verbindung (A) α-Tocopherol oder α-Tocopherolacetat ist.

7. Zusammensetzung (C₂), wie einem der Ansprüche 5 oder 6 definiert, **dadurch gekennzeichnet, dass** das Massenverhältnis zwischen der Verbindung (A) und der durch die Formel (I) dargestellten Zusammensetzung größer oder gleich 1/10 und kleiner oder gleich 6,5/10 ist.

8. Nicht-therapeutische Verwendung der Zusammensetzung (C₂), wie in einem der Ansprüche 5 bis 7 definiert, zur Verhinderung von unansehnlichen Auswirkungen der Alterung von Haut, Haaren, Kopfhaut oder Schleimhäuten.

9. Verwendung der Zusammensetzung (C₂), wie in einem der Ansprüche 5 bis 7 definiert, zur Herstellung einer Zusammensetzung, die zur Reinigung von Haut, Haaren, Kopfhaut oder Schleimhäuten bestimmt ist.

10. Zusammensetzung (C₂), wie in einem der Ansprüche 5 bis 7 definiert, zur Durchführung eines Verfahrens zur therapeutischen Behandlung des menschlichen oder tierischen Körpers zur Behandlung und/oder Verhinderung von sichtbaren Rötungen und/oder Reizungen der Haut und/oder Kopfhaut infolge von Sonnenstrahlung.

## Claims

1. Use of a composition represented by formula (1):
R-O-(G)ₚ-H (I)
in which R represents a radical chosen from n-heptyl or 2-ethyl hexyl radicals, G represents the residue of the glucose and p represents a decimal number greater than or equal to 1.05 and less than or equal to 5, said composition of formula (I) consisting of a mixture of compounds represented by formulae (I₁), (I₂), (I₃), (I₄) and (I₅):
R-O-(G)₁-H (I₁)
R-O-(G)₂-H (I₂)
R-O-(G)₃-H (I₃)
R-O-(G)₄-H (I₄)
R-O-(G)₅-H (I₅)
in respective molar proportions a₁, a₂, a₃, a₄, and a₅, such that:
- The sum a₁ + a₂ + a₃ + a₄ + a₅ is equal to 1 and that
- each one of the proportions a₁, a₂, a₃, a₄, and a₅ is greater than or equal to zero and less than or equal to one,
as solubilising agent in an aqueous composition of at least one compound (A) chosen from α-tocopherol, β-Tocopherol, γ-tocopherol, δ-tocopherol, α-tocopherol acetate, β-tocopherol acetate, γ-tocopherol acetate, δ-tocopherol acetate, α-tocotrienol, β-tocotrienol, γ-tocotrienol and δ-tocotrienol.

2. Use such as defined in claim 1, **characterised in that** in the formula (I), p represents a decimal number greater than or equal to 1.05 and less than or equal to 2.5.

3. Use such as defined in one or any of claims 1 or 2, **characterised in that** the compound (A) is α-tocopherol or α-tocopherol acetate.

4. Use such as defined in one or any of claims 1 to 3, **characterised in that** the weight ratio between the compound (A) and the composition represented by the formula (I), is greater than or equal to 1/10 and less than or equal to 6.5/10.

5. Composition (C₂) comprising for 100% of its weight:
a) from 0.05% to 12.0% by weight of at least one composition represented by the formula (I) such as defined in claim 1;
b) from 0.01 to 5.0% by weight of at least one compound (A) chosen from α-tocopherol, β-Tocopherol, γ-tocopherol, δ-tocopherol, α-tocopherol acetate, β-tocopherol acetate, γ-tocopherol acetate, δ-tocopherol acetate, α-tocotrienol, β-tocotrienol, γ-tocotrienol and δ-tocotrienol.
c) from 83.0% to 99.94% by water weight.

6. Composition (C₂) such as defined in claim 5, **characterised in that** the compound (A) is α-tocopherol or α-tocopherol acetate.

7. Composition (C₂) such as defined in one of claims 5 or 6, **characterised in that** the weight ratio between the compound (A) and the composition represented by the formula (I), is greater than or equal to 1/10 and less than or equal to 6.5/10.

8. Non-therapeutic use of the composition (C₂) such as defined in any of claims 5 to 7, in order to prevent the unaesthetic effects of the ageing of the skin, hair, scalp or of the mucosa.

9. Use of the composition (C₂) such as defined in one of claims 5 to 7 for the preparation of a composition intended for the cleaning of the skin, hair, scalp or mucosa.

10. Composition (C₂) such as defined in one of claims 5 to 7 for the implementing of a method for the therapeutic treating of the human or animal body intended to treat and/or prevent visible redness and/or irritations of the skin and/or of the scalp due to the rays of the sun.
